(19) Europäisches Patentamt
European Patent Office
Office européen des brevets

(11) Veröffentlichungsnummer : **0 173 120**
**B1**

(12) # EUROPÄISCHE PATENTSCHRIFT

(45) Veröffentlichungstag der Patentschrift : **02.03.88**

(51) Int. Cl.[4] : **C 07 C149/23**, C 07 C149/41, C 07 C149/437, C 08 K 5/37

(21) Anmeldenummer : **85109815.2**

(22) Anmeldetag : **05.08.85**

(54) **Alterungsschutzmittel und diese enthaltende Polymere.**

(30) Priorität : **18.08.84 DE 3430510**

(43) Veröffentlichungstag der Anmeldung : **05.03.86 Patentblatt 86/10**

(45) Bekanntmachung des Hinweises auf die Patenterteilung : **02.03.88 Patentblatt 88/09**

(84) Benannte Vertragsstaaten : **DE FR GB IT**

(56) Entgegenhaltungen :
**EP-A- 0 101 785**
**EP-A- 0 130 522**
**FR-A- 1 571 696**
**FR-A- 2 361 424**
**GB-A- 1 441 621**

(73) Patentinhaber : **BAYER AG**
**Konzernverwaltung RP Patentabteilung**
**D-5090 Leverkusen 1 Bayerwerk (DE)**

(72) Erfinder : **Buysch, Hans-Josef, Dr.**
**Brandenburger Strasse 28**
**D-4150 Krefeld (DE)**
Erfinder : **Witte, Josef, Dr.**
**Haferkamp 10**
**D-5000 Koeln 80 (DE)**
Erfinder : **Szentivanyi, Zsolt, Dr.**
**Carl-Rumpff-Strasse 9**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Verbindungen der Formel (I)

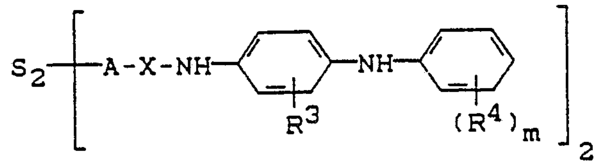

(I).

worin

$R^3$ H,

$R^4$ $C_{7-13}$-Aralkyl,

A einen zweibindungen Rest der Struktur

$-(CH_2)_n-$, $-(CH_2)_n-O-$, 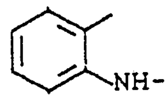

X —CO—,

m 1 oder 2 und

n eine ganze Zahl von 1 bis 5 bedeuten,

ihre Herstellung sowie Polymere, die diese Verbindungen als Akterungsschutzmittel enthalten.

Polymere werden bei Einwirkung von Licht, Luft und Hitze rasch verändert und verlieren in Folge von Abbau- und Vernetzungsvorgängen ihre guten Gebrauchseigenschaften. Daher werden den Polymeren Alterungsschutzmittel zugesetzt, die ihre Lebensdauer beträchtlich verlängern. Eine Zumischung von Alterungsschutzmitteln reicht aber häufig nicht aus, wenn Gebrauchsartikel aus solchen Polymeren mit Medien in Berührung kommen, die die Alterungsschutzmittel zu extrahieren vermögen und damit ihre Schutzfunktion erheblich beeinträchtigen oder gar aufheben. Es ist auch bekannt, daß Alterungsschutzmittel migrieren und aus den Polymeren ausschwitzen.

Man hat vorgeschlagen, in solchen Fällen die Alterungsschutzmittel an das Polymere zu binden oder Polymere Alterungsschutzmittel zu verwenden.

So wird in der DE-OS 2 735 178 beschrieben, an die Polymerkette Alterungsschutzmittel der allgemeinen Formel

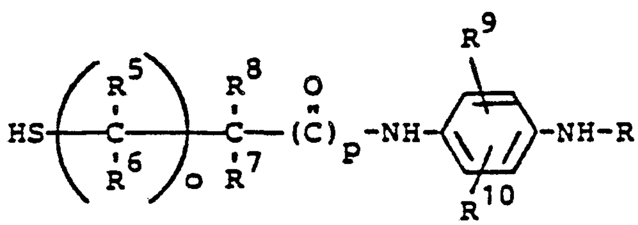

(II)

worin

R unter anderem gegebenenfalls durch $C_1$-$C_4$-Alkyl substituiertes Phenyl

$R^5$-$R^{10}$ H oder $C_1$-$C_5$-Alkyl,

P 0 oder 1 und

o 0 bis 12 bedeuten, zu knüpfen.

Zwar läßt sich nachweisen, daß diese Alterungsschutzmittel am Polymeren zumindestens teilweise fixiert werden und unter extraktiven Alterungsbedingungen einen gewissen begrenzten Alterungsschutz gewähren, doch besitzen so ausgerüstete Polymere eine merklich geringere Alterungsbeständigkeit, als sie mit bekannten, nicht fixierten Alterungsschutzmitteln auf Aminbasis wie Distyryldiphenylamin erreicht werden kann.

Ein weiterer Nachteil besteht darin, daß sie die Anvulkanisationsdauer (Scorchtime) und damit die Verarbeitungszeit der sie enthaltenden Polymermassen empfindlich einschränken.

Aufgabe der Erfindung war es, Alterungsschutzmittel bereitzustellen, die sowohl eine hinreichende Verarbeitungszeit garantieren, eine gute Schutzwirkung bieten, als auch an Polymere angebunden werden können und damit unter extraktiven Bedingungen gut wirksam bleiben.

Diese Aufgabe wird durch die eingangs beschriebenen Verbindungen I gelöst, worin bevorzugt

$R^3$ H,

$R^4$ $C_{7-13}$-Aralkyl,

A einen zweibindungen Rest der Struktur

$-(CH_2)_n-$, $-(CH_2)_n-O-$, 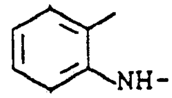

X —CO—,
m 1 oder 2 und
n eine ganze Zahl von 1 bis 5 bedeuten.

Die erfindungsgemäßen Verbindungen können wird nach verschiedenen Verfahren hergestellt werden. Im ersten Verfahren setzt man 4-Aminodiphenylaminderivate der Formel III

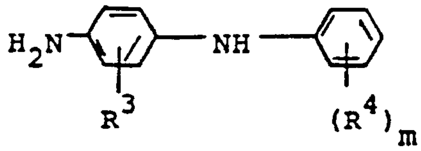

(III)

mit Verbindungen der Formel IV

$$S_2-[A-X-Y]_2 \qquad (IV)$$

um, worin $R^3$, $R^4$, m, A und X die vorstehend genannte Bedeutung haben und Y für Cl oder $OR^5$ stehen und $R^5$, H, $C_{1-4}$ Alkyl oder Aryl bedeutet.

Die erfindungsgemäßen Verbindungen können auch durch Oxidation von Mercaptoverbindungen der Formel V

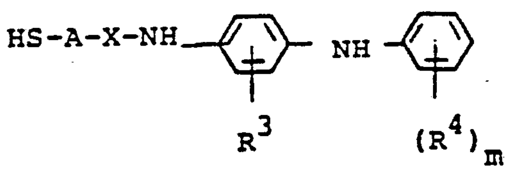

(V)

hergestellt werden.

Die Oxidation der Mercaptoverbindungen wird nach an sich bekannten Methoden mit Halogenen, Peroxiden oder Luftsauerstoff durchgeführt.

In einem weiteren Verfahren werden Verbindungen der Formel VI

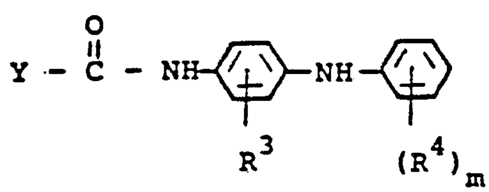

(VI)

mit Verbindungen der Formel VII

$$S_2-[A-H]_2 \qquad (VII)$$

in molaren Verhältnis 2 : 1 umgesetzt.

Die erfindungsgemäßen Verbindungen können durch Umkristallisieren gegebenenfalls in Gegenwart von Adsorbentien gereinigt werden. In vielen Fällen erübrigt sich dieser Schritt, und man setzt Rohprodukte ein.

Die neuen Alterungsschutzmittel der Formel (I) können auf mehreren Wegen an Polymere angebunden werden, nämlich während der radikalischen Polymerisation der unten genannten Monomeren, vorzugsweise durch Addition an fertige Polymerisate, besonders bevorzugt aber während der Härtung und Vulkanisation der Polymeren.

Diese Reaktionen werden nach an sich bekannten Verfahren in Gegenwart der Verbindungen I in Masse, Emulsion, Lösung oder Dispersion, die Härtung oder Vulkanisation unter den üblichen Bedingungen und in Anwesenheit der bekannten Härtungs- und Vulkanisationssysteme durchgeführt.

Die Menge an Alterungsschutzmittel beträgt 0,2-10 Gew.-%, bevorzugt 0,5-5 Gew.-% bezogen auf das Polymere.

Die erfindungsgemäßen Alterungsschutzmittel I können auch an Polymere mit Molgewichten von 1 000 bis 30 000 (Zahlenmittel), vorzugsweise von 2 000 bis 20 000 in hoher Konzentration addiert werden, so daß die Polymere einen Gehalt von 10 bis 60 Gew.-%, vorzugsweise von 15 bis 50 Gew.-% an gebundenem Alterungsschutzmittel enthalten. Derartige Verbindungen werden dann den hochmolekularen Polymeren zugesetzt und bilden ebenfalls migrationsfeste und schwer extrahierbare wirksame Polymere Alterungsschutzmittel. Sie werden in solchen Mengen den hochmolekularen Polymeren zugesetzt, daß die vorstehend genannten Konzentrationen an Alterungsschutzmittel im Gesamtpolymer erhalten werden. Dazu werden die das Alterungsschutzmittel gebunden enthaltenden Polymeren mit niederem Molgewicht in Mengen von 1-25 bevorzugt 4-20 Gew.-%, bezogen auf die hochmolekularen Polymeren, eingesetzt.

Geeignete Polymere mit niedrigen Molgewichten für derartige Additionsreaktionen sind beispielsweise Polybutadiene, Polyisoprene, Mischpolymere von Butadien und/oder Isopren mit Styrol, Acrylnitril, Methylmethacrylat, Ethylacrylat, α-Methylstyrol, Piperylen, Hexadien-1,3, Ethylen, Propylen und Vinylacetat.

Die Addition der Alterungsschutzmittel an die Polymere kann unter radikalischen Bedingungen, beispielsweise in Gegenwart bekannter Radikalstarter wie Dicumylperoxid, Di-tert.-butylperoxid oder Azodiisobutyronitril erfolgen.

Bevorzugt wird sie jedoch rein thermisch bei Temperaturen oberhalb 100 °C, vorzugsweise 120-300 °C, besonders bevorzugt 140-280 °C durchgeführt, wobei die Addition glatt und in hohen Ausbeuten verläuft.

Die neuen Alterungsschutzmittel eignen sich für eine breite Palette von Kautschuken und Kunststoffen, insbesondere aber für Kautschuke, z. B. für Polymere aus 1,3-Dienen wie Butadien, Isopren, Piperylen, 2-Chlorbutadien, 2-Ethyl-butadien und deren Copolymere mit Vinylmonomeren, wie Styrol, p-Methylstyrol, α-Methylstyrol, Norbornen, Norbornadien, Acrylsäure, Acrylsäureester und -amide, Acrylnitril, Ethylen, Propylen und Vinylacetat, für Polyalkenamere, beispielsweise aus Cyclopenten oder 1,5-Cyclooctadien, und für Polymere aus 1-Olefinmischungen, beispielsweise aus Ethylen/Propylen oder Ethylen/Propylen/Dien mit isolierten Doppelbindungen. Solche Polymeren können durch radikalische, koordinative, metathetische oder ionische Polymerisation entstanden sein.

Beispiele derartiger Polymere sind : BR, Naturkautschuk, SBR, NBR, EPDM und CR, Polypentenamer, ferner Polyethylen, Polypropylen oder Polystyrol mit geringen Gehalten and Doppelbindungen, schließlich auch ein- und mehrphasige Polymermischungen, wie ABS oder Polystyrol, Polyethylen, Polypropylen, bevorzugt aber Doppelbindungen enthaltende Polymerisate.

Besonders wirkungsvoll sind die Alterungsschutzmittel bei Nitrilkautschuk.

Die Kautschuke können vulkanisiert sein.

Eine weitere Verbesserung der Bruchdehnung kann durch Zusatz von 5 bis 15 Gew.-%, bezogen auf Kautschukfeststoff, oligomeren Thioether, beispielsweise Etherthioether wie Vulkanol 85® (Bayer AG, Leverkusen), erzielt werden.

Beispiel 1

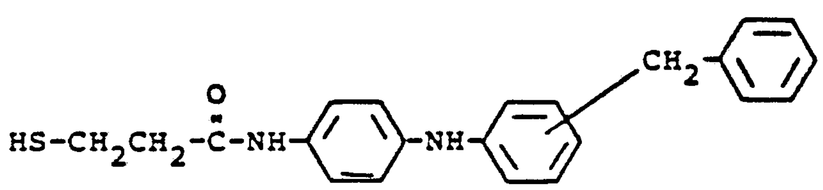

a) Zu einem Gemisch von 184 g (1,0 Mol) 4-Aminodiphenylamin und 20 g säureaktivierter Bleicherde wurden unter Rühren und Stickstoff bei 200 °C 216 g (2 Mol) Benzylalkohol in 1 h zugetropft. Man hielt noch 3 h bei 200 °C, filtrierte nach Verdünnen mit Toluol durch eine Drucknutsche, dampfte das Filtrat ein und destillierte unter vermindertem Druck nicht umgesetzte Ausgangsprodukte und schließlich bei 230-260 °C/0,6-1,0 mbar 280 g einer Fraktion, die neben dem benzylsubstituierten 4-Aminodiphenylamin geringe Mengen der dibenzylsubstituierten Verbindung enthielt.

b) Ein Gemisch von 137 g (ca. 0,5 Mol) des Produktes aus 1a, 53 g (0,5 Mol) β-Mercaptopropionsäure und 250 ml Xylol wurden am Wasserabscheider unter Rückfluß, Stickstoff und Rühren gekocht, bis kein Wasser mehr abgeschieden wurde. Insgesamt konnten 7,3 ml $H_2O$ abgetrennt werden. Die Lösung wurde bis zu einer Sumpftemperatur von 175 °C/10 mbar eingedampft und damit von Xylol und nicht umgesetzter Mercaptopropionsäure befreit. Man erhielt 168 g eines braunen Harzes mit einem SH-Gehalt von 8,4 %, d. h. 92 % des errechneten Wertes.

Beispiel 2

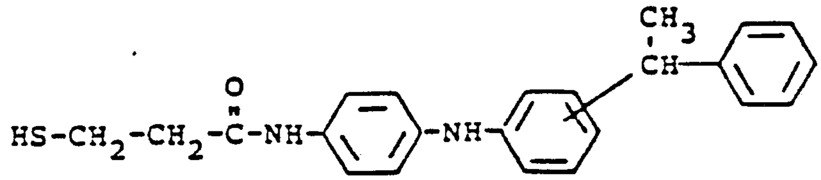

a) Zu einem Gemisch von 184 g (0,1 Mol) 4-Aminodiphenylamin und 20 g säureaktivierter Bleicherde wurden bei 200 °C unter Rühren und Stickstoff in 2 h 208 g (2,0 Mol) Styrol zugetropft und das Gemisch noch 1 h bei 200 °C gehalten. Nach Verdünnen mit Toluol wurde durch eine Drucknutzsche filtriert, das Filtrat unter vermindertem Druck destilliert und von Lösungsmittel und nicht umgesetzten Ausgangsprodukten befreit. Schließlich gingen bei 220-243 °C/0,2-0,3 mbar 286 g der gewünschten Verbindung über.

b) Ein Gemisch von 144 g (ca. 0,5 Mol) der Verbindung aus 2a, 53 g (0,5 Mol) β-Mercaptopropionsäure und 250 ml Xylol wurde unter Rühren, Rückfluß und Stickstoff am Wasserabscheider gekocht, bis 7,5 ml Wasser abgetrennt werden. Dann wurde die Lösung bis zu einer Sumpftemperatur von 170 °C bei 10 mbar eingedampft. Man erhielt 176 g eines braunen Harzes mit einem SH-Gehalt von 8,3 % entsprechend 94 % des errechneten Wertes.

## Beispiel 3

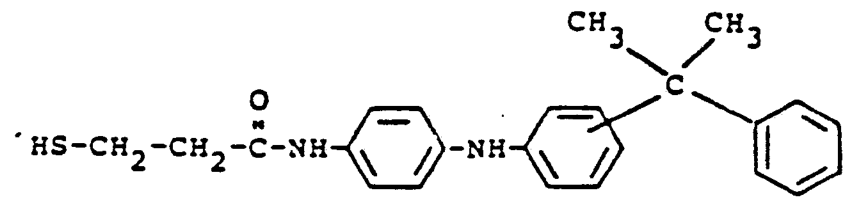

und

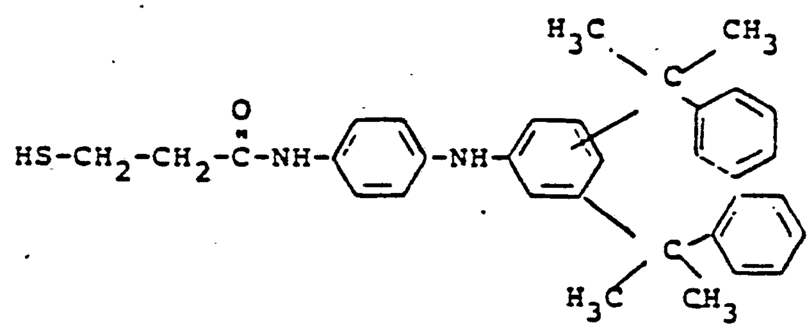

a) Zu einem Gemisch von 800 g (4,35 Mol) 4-Aminodiphenylamin und 80 g säureaktivierter Bleicherde tropfte man unter Rühren und Stickstoff bei 200 °C in 2 h 1 025 g (8,7 Mol) α-Methylstyrol, hielt noch 1 h bei 200 °C, filtrierte das mit Toluol verdünnte Reaktionsgemisch durch eine Drucknutsche und destillierte das Filtrat unter vermindertem Druck, wobei zunächst Lösungsmittel und nicht umgesetzte Ausgangsprodukte übergingen, dann folgte bei 236-255 °C/0,3 mbar eine Fraktion I (787 g), die im wesentlichen aus monoalkylierten Verbindungen bestand und dann bei 268-288 °C/0,3-0,5 mbar eine Fraktion II (530 g), die im wesentlichen aus der dialkylierten Verbindung bestand.

b) Ein Gemisch aus 151 g (ca. 0,5 Mol) der Fraktion I aus 3a, 53 g (0,5 Mol) β-Mercaptopropionsäure und 250 ml Xylol ließ man wie in 2b bis zur Beendigung der Wasserabscheidung (8,0 ml) reagieren und arbeitete analog auf. Man erhielt ein langsam kristallisierendes braunes Harz (188 g) mit einem SH-Gehalt von 8,4 %, entsprechend dem berechneten Wert.

## Beispiel 4

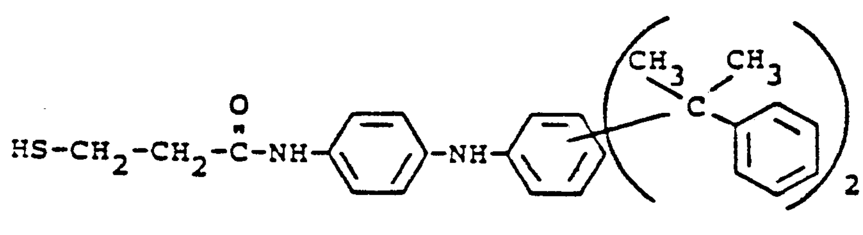

Ein Gemisch aus 210 g (ca. 0,5 Mol) der Fraktion II aus 3a, 53 g (0,5 Mol) β-Mercaptopropionsäure und 300 ml Xylol wurden die in 3b miteinander umgesetzt. Man erhielt 7,5 ml $H_2O$ und ein dunkelbraunes Harz mit einem SH-Gehalt von 6,1 % entsprechend 97 % des berechneten Wertes.

Beispiel 5

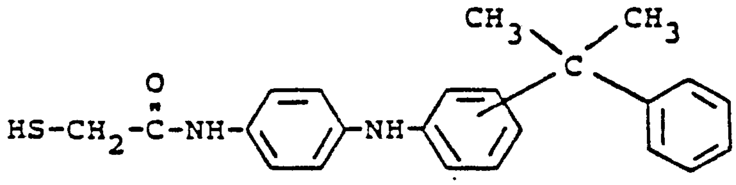

Ein Gemisch von 151 g (ca. 0,5 Mol) der Fraktion I aus 3a, 46 g (0,5 Mol) Mercaptoessigsäure und 250 ml Xylol wurden wie in 3b miteinander umgesetzt, wobei 8,5 ml $H_2O$ abgespalten wurden. Man erhielt 188 g eines hellbraunen, langsam kristallisierenden Harzes mit einem SH-Gehalt von 7,8 %, das sind 89 % des berechneten Wertes.

Beispiel 6

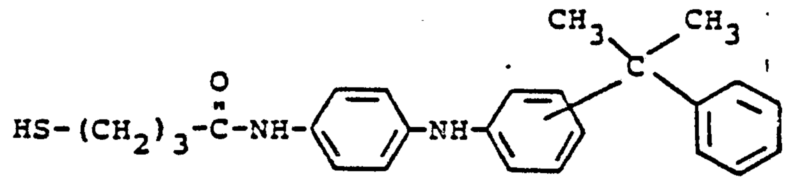

Ein Gemisch aus 151 g (ca. 0,5 Mol) der Fraktion I aus 3a, 51 g (0,5 Mol) γ-Thio-butyrolacton wurden 5 h unter Rühren und Stickstoff auf 140-150 °C erhitzt. Das gebildete hellbraune Harz wies einen SH-Gehalt von 7,3 % auf, das sind 89 % des berechneten Wertes.

Beispiel 7

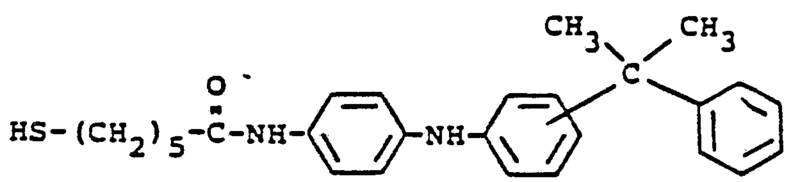

Ein Gemisch aus 151 g (ca. 0,5 Mol) der Fraktion I aus 3a, 74 g (0,5 Mol) ε-Mercapto-capronsäure und 300 ml Xylol wurden wie in 3b miteinander umgesetzt und anschließend bis zu einer Sumpftemperatur von 180 °C bei 1 mbar von flüchtigen Substanzen befreit. Man erhielt ein braunes Harz mit einem SH-Gehalt von 7,3 %, das sind 95 % des berechneten Wertes.

Beispiel 8

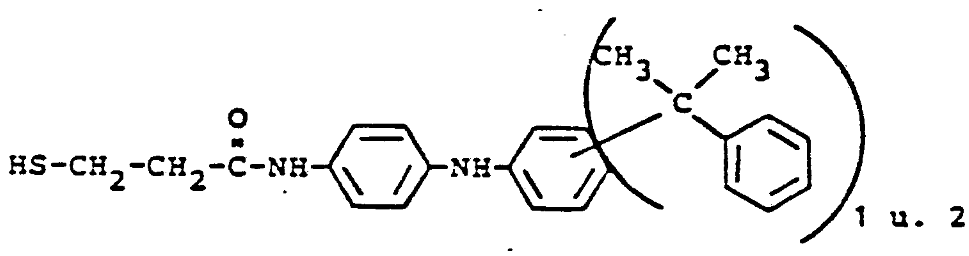

Beispiel 3a wurde wiederholt, das rohe Reaktionsprodukt wurde jedoch nicht fraktioniert destilliert, sondern 132 g davon wurden nach Abtrennung nicht umgesetzter Ausgangsprodukte direkt mit 41 g β-Mercaptopropionsäure kondensiert. Man erhält ein tiefdunkelbraunes Harz mit einem SH-Gehalt von 7,5 %.

Beispiel 9

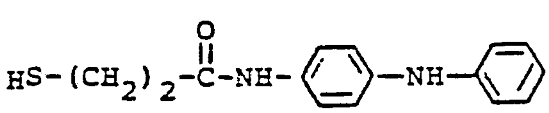

Ein Gemisch von 184 g (1 Mol) 4-Aminodiphenylamin, 106 g (1 Mol) β-Mercaptopropionsäure und 300 ml Xylol wurden unter Stickstoff und Auskreisen von Wasser gekocht bis 25-30 ml $H_2O$ abdestilliert

waren. Nach Abkühlen wurde abgesaugt und aus Toluol umkristallisiert : 151 g, Fp 96-98 °C, SH-Gehalt ber. 11,7 % gef. 11,5 %.

Beispiel 10

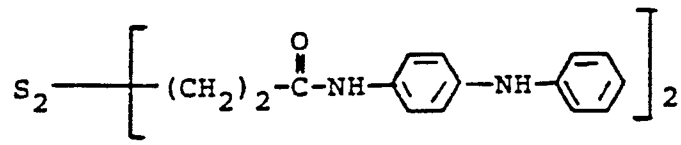

10 g β-Mercaptocarbonamid aus Beispiel 9 wurden in Butanol gelöst, mit 3,2 g 30 %igem Wasserstoffperoxid versetzt und 8 h bei 50 °C gerührt. Man erhielt 7,3 g vom Fp 191-194 °C.
S-Gehalt ber. 11,7 gef. 11,5 %
SH-Gehalt ber. 0 gef. 0,2 %

Beispiel 11

10 g β-Mercaptocarbonamid aus Beispiel 9 wurden in Butanol gelöst und 10 h bei 50 °C mit Luft Rühren begast. Nach Abkühlen wurde ein Produkt vom Fp 195-8 °C isoliert mit einem S-Gehalt von 11,7 % (ber. 11,7 %) und einem SH-Gehalt von 0,13 %.

Beispiel 12

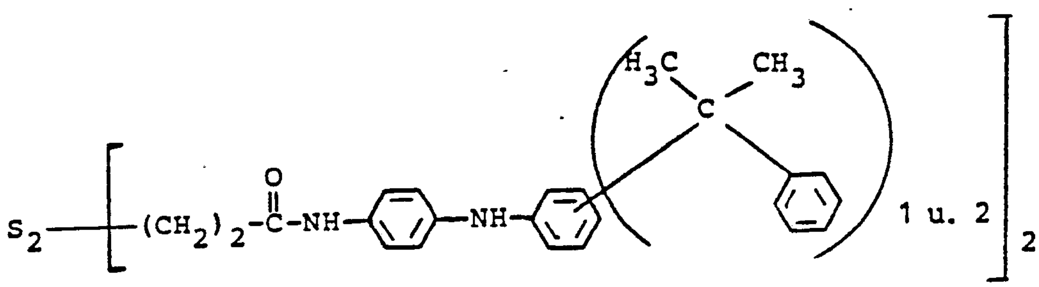

130 g des Produktes aus Beispiel 8 wurden in Ethanol gelöst mit 30 g 45 %iger Natronlauge unter Kühlung portionsweise versetzt. Zu dieser Lösung tropft man bei 20-25 °C in 4 h eine ethanolische Lösung von 38 g Iod. Nach weiteren 30′ wurde die Lösung unter vermindertem Druck eingeengt, der Rückstand in Wasser und Toluol aufgenommen, die Phasen getrennt, die organische mit Wasser gewaschen und eingedampft. Man erhielt ein dunkles Harz mit einem S-Gehalt von 6,9 % (ber. 7.5 %) und einem SH-Gehalt von 0,3 % (ber. 0 %).

Beispiel 13

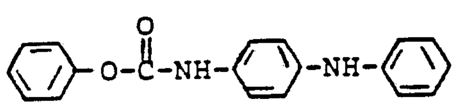

a) Zu einer Lösung von 184 g (1 Mol) 4-Aminodiphenylamin und 110 g (1,10 Mol) Triethylamin in Toluol wurde in 2 h bei 30-40 °C eine Lösung von 150 g (1 Mol) Chlorameisensäurephenylester unter Rühren und Stickstoff zugetropft. Nach weiteren 4 h bei 30 °C wurde abgesaugt, mit Toluol und dann mit Wasser gründlich gewaschen und getrocknet : 241 g (schmilzt unter Reaktion).

b)

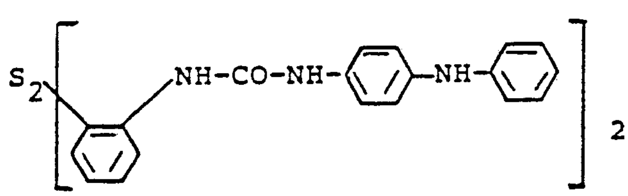

20 g o,o′-Diaminodiphenyldisulfid und 48,8 g des Urethans aus 13a) wurden in Dimethylacetamid 40-50′ auf 100 °C erhitzt und die Lösung in Methanol eingegossen. Die sich dabei abscheidenden dunklen Flocken wurden abgetrennt und in die klare Lösung Wasser eingetropft. Es schieden sich 39 g hellgrauer Kristalle ab. Fp. 119-122 °C.

Beispiel 14

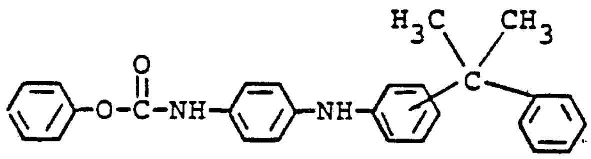

a) Beispiel 13a) wurde mit dem Amin aus Beispiel 3a, Fr. I statt mit 4-Aminodiphenylamin wiederholt. Man erhielt laut NMR-Analyse obiges Urethan Fp. 116-9 °C.

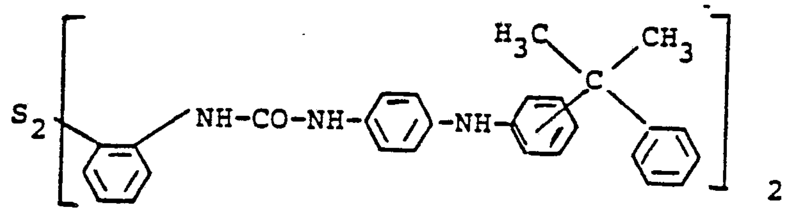

b) Beispiel 13b) wurde wiederholt mit Urethan aus 14a statt 13a. Man erhielt laut Analyse den obigen Harnstoff.

Beispiel 15

Ein Gemisch aus 60 Gew.-% eines NBR-Öles (MG = 1 900, 23 % Acrylnitril) und 40 Gew.-% des Disulfid-diamids aus Beispiel 12 wurde unter Stickstoff 10 min auf 230 °C erhitzt. Das modifizierte Öl wurde durch 3-faches Umfällen aus Toluol/Ethanol gereinigt. Die Ausbeute an umgefälltem Öl betrug 57 % bezogen auf die Summe der Einsatzprodukte. Der Schwefelgehalt des umgefällten modifizierten NBR-Öles betrug 2,9 %, entsprechend einer praktisch quantitativen Bindung des Produktes aus Beispiel 12 an das NBR-Öl.

Beispiel 16

Ein Gemisch aus 53 Gew.-% eines Polybutadienöles (MG = 2 100, 97 % 1,4-Anteil) und 47 Gew.-% des Produktes aus Beispiel 14 wurde wie in Beispiel 15 umgesetzt und durch Umfällen gereinigt. Man erhielt eine Ausbeute an umgefälltem modifizierten Polymeren von 70 %, bezogen auf die Summe an Augangsprodukten, dessen Schwefelgehalt 3,0 % betrug entsprechend einer ca. 90 %igen Bindung des Produktes aus Beispiel 14 an das Polybutadienöl.

Beispiel 17

Ein NBR-Kautschuk aus 72 % Butadien und 28 % Acrylnitril wurde nach folgender Rezeptur in Anwesenheit von Stabilisatoren vulkanisiert :

| | |
|---|---|
| 100,00 Gew.-Teile | NBR |
| 0,75 Gew.-Teile | Stearinsäure |
| 3,0 Gew.-Teile | Zinkoxid |
| 1,5 Gew.-Teile | Mercaptosilan |
| 2,5 Gew.-Teile | eines Gemisches aus Fettsäure und Fettsäureestern |
| 30,0 Gew.-Teile | gefällte Kieselsäure |
| 30,0 Gew.-Teile | Kaolin calciniert |
| 0,25 Gew.-Teile | Schwefelgranulat 80 %ig |
| 2,5 Gew.-Teile | Tetramethylthiuramdisulfid |
| 2,0 Gew.-Teile | Dibenzothiazyldisulfid |
| 4,0 Gew.-Teile | Mercaptobenzthiazol-Zinksalz |
| 10,0 Gew.-Teile | Thioether |
| 2,0 Gew.-Teile | Alterungsschutzmittel A-D |

A = Distyryldiphenylamin
B = Vergleichsprodukt Nr. II aus der Übersicht auf S. 15 der DE-OS 2 735 178
C = Produkt aus Beispiel 10 erfindungsgemäß
D = Produkt aus Beispiel 12 erfindungsgemäß

| | A | B | C | D |
|---|---|---|---|---|
| Mooney Scorch 120°C (min) | 16,2 | 7 | 15,7 | 15,3 |
| Vulkameter $t_{10}$ (min) | 2,4 | 2,3 | 2,4 | 2,3 |
| 170°C $t_{70}$ (min) | 3,4 | 3,1 | 4,1 | 3,4 |
| Vulkanisation 20' 170°C | Normstab II | | | |
| Zugfestigkeit (MPa) | 19,4 | 18,3 | 19,9 | 17,6 |
| Bruchdehnung % | 585 | 630 | 545 | 565 |
| Härte RT Shore A | 59 | 55 | 57 | 59 |
| Heißluftalterung 150°C | Zellofen 3 und 5 Tage | | | |
| Restbruchdehnung nach 3 Tagen (%) | 92 | 70 | 81 | 89 |
| Restbruchdehnung nach 5 Tagen (%) | 63 | 42 | 50 | 59 |
| Alterung in Kraftstoff C 48 h 40°C, 48 h Rücktrocknung bei 40°C im Vakuum, dann Heißluftalterung 135°C Zellofen 7 und 11 Tage | | | | |
| Restbruchdehnung nach 7 Tagen (%) | 11 | 33 | 66 | 73 |
| Restbruchdehnung nach 11 Tagen (%) | 4 | 21 | 51 | 60 |

Aus den obigen Messungen ergibt sich :

1) Vergleichsprodukt B entsprechend DE-OS 2 735 178 bewirkt eine drastische Verkürzung der Anvulkanisationsdauer gegenüber dem bekannten Alterungsschutzmittel A.

2) Die erfindungsgemäßen Produkte C und D verkürzen die Anvulkanisationsdauer gegenüber A praktisch nicht.

3) B weist gegenüber dem handelsüblichen A einen erheblich schlechteren Alterungsschutz auf (rasher Abfall der Bruchdehnung) bei der normalen Heißluftalterung. Die Alterung in Kraftstoff C mit nachfolgender Heißluftalterung weist allerdings Vorteile für B gegenüber A auf.

4) Die erfindungsgemäßen Produkte C und D nähern sich bzw. erreichen praktisch den mit A erzielbaren Alterungsschutz bei der bloßen Heißluftalterung. Besser als A und auch B schneiden C und D bei der Alterung in Kraftstoff mit nachfolgender Heißluftalterung ab. Die Bruchdehnung liegt hier erheblich höher als bei B und besonders als bei A.

5) Die Werte für C und D bei bloßer Heißluftalterung unterscheiden sich wenig oder kaum von denen, die nach extraktiver Alterung gemessen wurden, so daß man auf einen hohen Prozentsatz an gebundenen Alterungsschutzmittel schließen muß.

Für B liegen die Werte nach extraktiver Alterung deutlich niedriger als nach Heißluftalterung, weil offenbar ein nicht vernachlässigbarer Teil des Alterungsschutzmittels ausgewaschen wurde.

**Patentansprüche**

1. Verbindungen der allgemeinen Formel

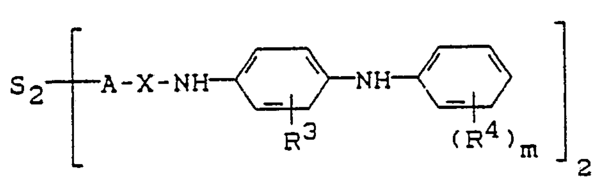

worin

$R^3$ H,

$R^4$ $C_{7-13}$-Aralkyl,

A einen zweibindungen Rest der Struktur

$-(CH_2)_n-$, $-(CH_2)_n-O-$, 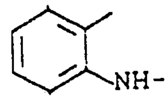

X —CO—,
m 1 oder 2 und
n eine ganze Zahl von 1 bis 5 bedeuten.

2. Polymere enthaltend Verbindungen nach Anspruch 1.

3. Kautschuke und Vulkanisate der Kautschuke enthaltend Verbindungen nach Anspruch 1.

4. Nitrilkautschuke und Vulkanisate von Nitrilkautschuken enthaltend Verbindungen nach Anspruch 1.

5. Polymere nach Anspruch 2, enthaltend 0,2 bis 10 Gew.-% an Verbindungen nach Anspruch 1.

**Claims**

1. Compounds corresponding to the following general formula

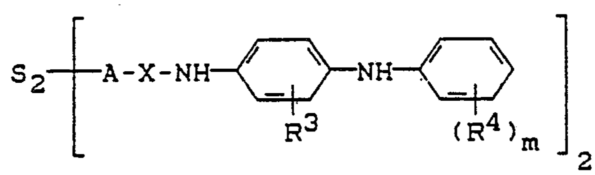

wherein
$R^3$ denotes H,
$R^4$ denotes $C_{7-13}$-aralkyl,
A denotes a 2-bonded group having a structure corresponding to one of the following formulae

$-(CH_2)_n-$, $-(CH_2)_n-O-$, 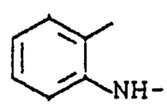

X denotes CO,
m represents 1 or 2 and
n represents an integer of from 1 to 5.

2. Polymers containing compounds according to claim 1.

3. Rubbers and vulcanizates of rubbers containing compounds according to claim 1.

4. Nitrile rubbers and vulcanizates of nitrile rubbers containing compounds according to claim 1.

5. Polymers according to claim 2 containing from 0.2 to 10 % by weight of compounds according to claim 1.

**Revendications**

1. Composés de formule générale

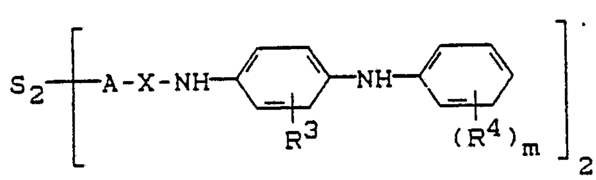

dans laquelle
$R^3$ représente H,
$R^4$ est un groupe aralkyle en $C_7$ à $C_{13}$,
A est un reste divalent de structure

$-(CH_2)_n-$, $-(CH_2)_n-O-$, 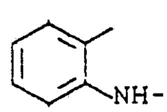

X représente —CO—,
m a la valeur 1 ou 2 et
n est un nombre entier de 1 à 5.

2. Polymère contenant des composés suivant la revendication 1.

3. Caoutchoucs et produits de vulcanisation des caoutchoucs contenant des composés suivant la revendication 1.

4. Caoutchoucs nitriliques et vulcanisats de caoutchoucs nitriliques contenant des composés suivant la revendication 1.

5. Polymères suivant la revendication 2, contenant 0,2 à 10 % en poids de composés suivant la revendication 1.